# FASCICULE DE BREVET EUROPEEN

(11) **EP 4 472 728 B1**
(45) Date de publication et mention de la délivrance du brevet: **09.09.2026**
(21) Numéro de dépôt: 23702594.5
(22) Date de dépôt: 02.02.2023
(51) Int. Cl.: A61N 1/36, A61N 1/40, A61N 1/06, A61N 1/04

(54) **DISPOSITIF D'ÉLECTROTHÉRAPIE ASSOCIANT L'ÉLECTROSTIMULATION ET LA TÉCARTHÉRAPIE**
ELEKTROTHERAPIEVORRICHTUNG MIT KOMBINATION VON ELEKTROSTIMULATION UND TECAR-THERAPIE
ELECTROTHERAPY DEVICE COMBINING ELECTROSTIMULATION AND TECAR THERAPY

(30) Priorité: 03.02.2022 FR 2200974
(43) Date de publication de la demande: 11.12.2024
(73) Titulaire: Winback Group, 64200 Biarritz (FR)
(72) Inventeur: BUÉE, Christophe, 64500 Saint Jean de Luz (FR); JOVÈNE, Gilles, 06700 St Laurent du Var (FR); BROUCHET, Sebastien, 06310 Beaulieu sur Mer (FR)
(74) Mandataire: Hautier IP
(86) Numéro de dépôt international: PCT/EP2023/052602
(87) Numéro de publication internationale: WO 2023/148289

(56) Documents cités:
- WO-A2-2005/002663
- IT-U1- 201800 002 598
- KR-A- 20100 056 849
- US-A1- 2017 001 004

## Description

### DOMAINE TECHNIQUE

La présente description concerne un appareil électronique pour un usage thérapeutique ou cosmétique. Cet enseignement trouvera plus particulièrement son application dans le domaine dit de l'électrothérapie et notamment pour regrouper dans un canal deux familles de types de courants combinant avantageusement des basses fréquences, des moyennes fréquences (électrostimulation) et des hautes fréquences (Radiofréquence).

### ETAT DE LA TECHNIQUE

L'électrothérapie est une technique non invasive, sans danger qui consiste à employer de l'électricité dans un but thérapeutique. Cette technique est reconnue pour soulager la douleur, renforcer les fibres musculaires ou bien encore accélérer la cicatrisation des tissus biologiques. 3 grandes familles de fréquences existent comme les basses fréquences (1 Hz-150 Hz) pour une action neurostimulante superficielle, les moyennes fréquences (1 kHz - 10 kHz) pour une action neurostimulante profonde et enfin les hautes fréquences (100 kHz -1,2 MHz) pour une action diathermique sélective superficielle ou profonde. Les basses fréquences (BF), moyennes fréquences (MF) sont désignées généralement comme électrostimulation et les hautes fréquences (HF) sont désignées comme radiofréquences.

Ces différents types de courants d'électrothérapie circulent entre deux plaques qui jouent le rôle d'électrodes au contact de la peau et qui sont fixes ou mobiles. Les différents types de courants sont utilisés d'une manière séquentielle par le praticien. En basse fréquence (1Hz-150Hz), le courant désigné « micro courant » se compose de train d'impulsions pour le traitement des douleurs chroniques, des inflammations, du drainage et du recrutement musculaire. En général, il se caractérise par des impulsions dont les caractéristiques électriques sont nettement inférieures aux autres formes de courants en électrostimulation. Le micro courant s'avère particulièrement indiqué pour des zones localisées et superficielles.

En moyenne fréquence (1 kHz et 10 kHz), le courant désigné interférentiel est de type sinusoïdal modulé par un signal basse fréquence et est en mesure de pénétrer en profondeur avec une bonne tolérance de la part du patient. Il se compose d'une porteuse sinusoïdale entre 1 kHz et 10 kHz avec une modulation d'amplitude pour créer un signal de basse fréquence portée qui permet de contracter les muscles profonds avec une action antalgique. Ce courant est particulièrement utilisé pour recruter les muscles profonds essentiels à la posture du patient. On connait donc des appareils d'électrostimulation pour le renforcement musculaire utilisés soit de manière autonome, soit par un praticien tel qu'un kinésithérapeute.

Par ailleurs, la chaleur est une modalité thérapeutique utilisée depuis de nombreuses années en kinésithérapie et divisée en deux catégories : les agents chauffants superficiels et les agents chauffants profonds. Les modalités de thermothérapie profonde incluent la diathermie thérapeutique à ondes longues et à ondes courtes, les ultrasons et la radiofréquence de contact, cette dernière étant appelée aussi courant de haute fréquence qui est entre 100 kHz et 1,2 MHz. Ce type de thermothérapie profonde est appelé diathermie.

On connait également la técarthérapie basée sur le courant TECAR de l'anglais Transfert Energy Capacitive And Resistive, ou en français le transfert d'énergie capacitive et résistive. La técarthérapie est un traitement par radiofréquence de contact. C'est un type d'électrothérapie qui utilise les courants de haute fréquence. Le développement de cette technologie a été permis par le développement au début des années 90 des électrodes résistives. La técarthérapie génère un courant de radiofréquence de contact dans le spectre des hautes fréquences. Dans le mode capacitif, la résonance sera supérieure dans les tissus mous tels que les muscles tandis que dans le mode résistif, la résonance sera supérieure en profondeur dans les tissus durs tels les ligaments ou bien les os. Classiquement, le spectre de longueurs d'onde thérapeutiques va de 300 kHz à 1,2 MHz.

Il existe donc de nombreuses technologies au service du traitement thérapeutique par l'électricité, toutefois, chaque traitement doit être réalisé séparément avec des appareils séparés ce qui complique les protocoles de traitement, limite l'accès aux différentes technologies lorsque le praticien doit s'équiper d'une machine spécifique pour chaque application.

Il existe donc le besoin de proposer une solution technologique qui permet des traitements optimisés et simplifiés.

Les autres objets, caractéristiques et avantages du présent enseignement apparaîtront à l'examen de la description suivante et des dessins d'accompagnement. Il est entendu que d'autres avantages peuvent être incorporés. Les documents KR 2010 0056849, IT 2018 0000 2598 et WO 2005/002663 décrivent différents systèmes illustrant l'état de la technique.

### RESUME

Pour atteindre cet objectif, selon un mode de réalisation du présent enseignement, on prévoit un dispositif d'électrothérapie comprenant un premier module générateur de tension configuré pour générer une première tension ayant une première fréquence et comprenant une première borne de raccordement et une deuxième borne de raccordement, et un deuxième module générateur de tension configuré pour générer une deuxième tension ayant une deuxième fréquence strictement supérieure à la première fréquence et comprenant une troisième borne de raccordement et une quatrième borne de raccordement, ledit dispositif comprenant un canal d'émission, un canal de réception, et dans lequel la première borne de raccordement et la troisième borne de raccordement sont reliées audit canal d'émission et la deuxième borne de raccordement et la quatrième borne de raccordement sont reliées audit canal de réception.

Le présent enseignement permet d'appliquer deux tensions de deux fréquences différentes par un seul et même canal d'émission et un seul et même canal de réception.

Plus particulièrement, le dispositif d'électrothérapie comprend une unité de commande configurée pour commander le premier module générateur de tension pour générer la première tension à une première fréquence, commander le deuxième module générateur de tension la deuxième tension à une deuxième fréquence de manière simultanée.

Avantageusement, l'unité de commande est configurée pour générer dans le canal d'émission, un signal associant une tension sinusoïdale d'une première fréquence modulée par une troisième fréquence et à une tension sinusoïdale d'une deuxième fréquence modulée par une quatrième fréquence, le signal étant la somme de la tension du premier module de générateur et de la tension du deuxième module de générateur.

Un autre aspect non-revendiqué concerne un procédé pour le fonctionnement d'un dispositif d'électrothérapie tel que décrit ci-dessus comprenant :
a. la génération d'une première tension à une première fréquence par le premier module générateur, et
b. la génération d'une deuxième tension à une deuxième fréquence strictement supérieure à la première fréquence par le deuxième module générateur, les étapes a et b étant simultanées. Ainsi, les deux générateurs de tensions fonctionnent simultanément. La présente invention est définie par les revendications jointes ci-après.

### BREVE DESCRIPTION DES FIGURES

Les buts, objets, ainsi que les caractéristiques et avantages du présent enseignement ressortiront mieux de la description détaillée d'un mode de réalisation de cette dernière qui est illustré par les dessins d'accompagnement suivants dans lesquels :
La figure 1 représente le schéma du circuit électrique du dispositif selon le présent enseignement.
La figure 2A représente la tension moyenne fréquence sinusoïdale générée par le premier module de générateur.
La figure 2B représente le signal unitaire basse fréquence sinusoïdal généré par le premier module de générateur.
La figure 2C représente la tension sinusoïdale moyenne fréquence modulée par des basses fréquences générées par le premier module de générateur.
La figure 3A représente la haute fréquence sinusoïdale générée par le deuxième module de générateur.
La figure 3B représente le signal unitaire basse fréquence carrée généré par le deuxième module de générateur, plus précisément par la commande d'activation.
La figure 3C représente un signal sinusoïdal haute fréquence modulé par des basses fréquences à impulsion générées par le deuxième module de générateur.
La figure 4 représente le signal sinusoïdal moyenne fréquence modulé par des basses fréquences associé au signal sinusoïdal haute fréquence modulé par des basses fréquences dans le canal d'émission lors d'une application simultanée des deux courants.

Les dessins sont donnés à titre d'exemples et ne sont pas limitatifs de la présente description. Ils constituent des représentations schématiques de principe destinées à faciliter la compréhension du présent enseignement et ne sont pas nécessairement à l'échelle des applications pratiques.

### DESCRIPTION DÉTAILLÉE

Avant d'entamer une revue détaillée des modes de réalisation illustrant le présent enseignement, sont énoncées ci-après des caractéristiques optionnelles qui peuvent éventuellement être utilisées en association ou alternativement.

Selon un exemple, le premier module générateur de tension 100 est configuré pour générer une tension sinusoïdale.

Selon un exemple, la première fréquence est comprise dans un premier intervalle de fréquence entre 1 kHz à 10 kHz.

Selon un exemple, le premier module générateur de tension 100 est configuré pour générer une tension sinusoïdale de première fréquence modulée par une troisième fréquence.

Selon un exemple, la troisième fréquence est comprise dans un troisième intervalle de fréquence compris entre 1 Hz et 150 Hz.

Selon un exemple, la troisième fréquence est une tension sinusoïdale.

Le présent enseignement propose de créer un signal sinusoïdal de Moyenne Fréquence afin de moduler le signal de Basse Fréquence (électrostimulation) et de le combiner avec un signal sinusoïdal de Haute Fréquence (diathermie). Ceci permet de combiner au sein d'un unique et même canal les vertus de l'électrostimulation et de la radiofréquence générant la diathermie Selon le présent enseignement, il est produit un courant non invasif stimulant les mécanismes de cicatrisation naturels du corps et favorisant l'échange cellulaire. Ceci offre d'excellents résultats de réadaptation grâce à une récupération rapide des fonctions musculaires et articulaires.

Le résultat est un courant sinusoïdal de 1 kHz à 10 kHz modulé à une fréquence de 1 Hz à 150 Hz. En électrostimulation, la modulation du courant stimulateur permet d'éviter la tétanie des muscles excités.

Selon un exemple, le deuxième module générateur de tension 200 est configuré pour générer une tension sinusoïdale.

Selon un exemple, la deuxième fréquence est comprise dans un deuxième intervalle de fréquence compris entre 100 kHz à 10 MHz.

Selon un exemple, le deuxième module générateur de tension 200 est configuré pour générer une tension sinusoïdale de deuxième fréquence modulé par une quatrième fréquence.

Selon un exemple, la quatrième fréquence est comprise dans un quatrième intervalle de fréquence compris entre 1 Hz et 150 Hz.

Selon un exemple, la quatrième fréquence est à impulsion.

Selon un exemple, le deuxième module de générateur de tension 200 comprend une commande d'activation 38 configurée pour générer une tension sinusoïdale à impulsion à la quatrième fréquence.

Selon un exemple, le premier module de générateur de tension 100 comprend une commande d'émission 37 configurée pour émettre la tension du premier module de générateur à la quatrième fréquence.

Selon un exemple, le premier module de générateur 100 comprend un premier transformateur 11 présentant une première inductance et le deuxième module de générateur 200 comprend un deuxième transformateur 21 présentant une deuxième inductance, de préférence le rapport de la première inductance et de la deuxième inductance étant supérieure à 500, préférentiellement supérieure à 1000.

Selon un exemple, le deuxième module de générateur 200 comprend un filtre de sortie 24, avantageusement associé à un deuxième transformateur 21 comprenant un circuit LC comprenant une bobine 26 (L) et un condensateur 25 (Capacité),

Selon un exemple, le filtre de sortie 24 comprend un condensateur résonant 27 permettant de résonner à la deuxième fréquence du deuxième générateur.

Selon un exemple, l'unité de commande est configurée pour faire varier l'impédance dans le canal d'émission 4 lorsqu'elle commande le premier module 100.

Selon un exemple, le procédé pour le fonctionnement d'un dispositif d'électrothérapie 1 dans lequel lorsque le premier module générateur 100 est actif, l'impédance présentée du dispositif varie. Ce qui crée une modulation de la tension sinusoïdale haute fréquence avec la tension moyenne fréquence modulée basse fréquence.

Selon un exemple, le procédé pour le fonctionnement d'un dispositif d'électrothérapie 1 lorsque le premier module générateur 100 est actif, le deuxième transformateur 21 est déconnecté. Sinon il y aurait un risque d'avoir un court-circuit en sortie du premier transformateur 11 ne permettant pas de générer un courant de sortie.

Selon un exemple, le procédé pour le fonctionnement d'un dispositif d'électrothérapie 1 dans lequel lorsque le deuxième module générateur 200 est actif, le premier transformateur 11 ne modifie pas le courant sinusoïdal en sortie du deuxième transformateur 21. L'impédance présentée est négligeable devant celle de l'utilisateur 30.

La présente description concerne un dispositif d'électrothérapie 1 comprenant deux modules de générateurs de tensions 100, 200. Avantageusement, les deux modules de générateurs de tensions sont isolés.

Avantageusement, le dispositif permet de combiner simultanément les courants interférentiels, moyenne fréquence, les courants técarthérapie, haute fréquence, préférentiellement avec impulsions pour simuler les micro-courants, basse fréquence.

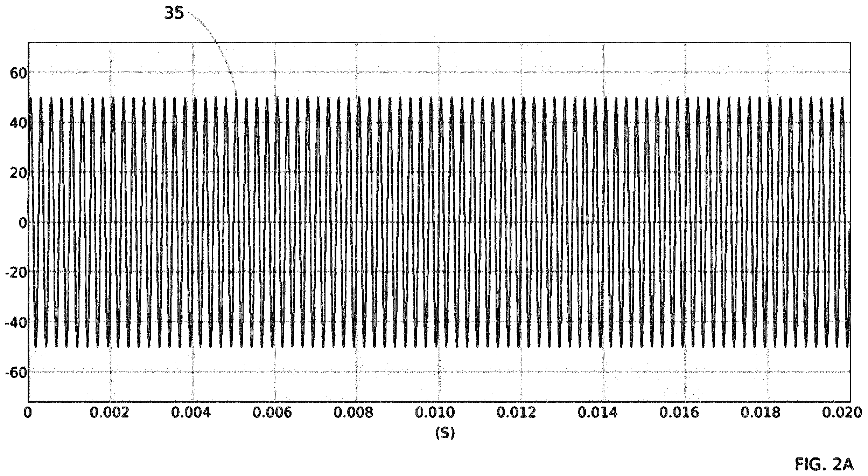

Le dispositif 1 comprend un premier module de générateur de tension 100 comprenant un premier générateur de tension 10. Le premier générateur de tension 10 est configuré pour générer une première tension ayant une première fréquence. Avantageusement, la première tension est une tension sinusoïdale. Préférentiellement, la première fréquence est comprise dans un premier intervalle de fréquence compris entre 1 kHz et 10 kHz. Cet intervalle de fréquence s'entend comme des fréquences de type moyennes fréquences. Un exemple de tension de moyennes fréquences sinusoïdales est illustré à la figure 2A. Cet intervalle de fréquence a notamment un effet drainant et participe à la récupération musculaire. Le premier module de générateur de tension 100 comprend une première borne de raccordement 12 et une deuxième borne de raccordement 13. Ce premier module générateur 100 est utilisé pour des applications d'électrostimulation et/ou pour un renfort musculaire en profondeur.

Le générateur 100 développe avantageusement un courant sinusoïdal de moyenne fréquence entre 1 kHz et 10 kHz préférentiellement avec une modulation d'amplitude basse fréquence pour stimuler un recrutement musculaire en profondeur et confortable.

Selon un mode de réalisation préféré, le dispositif, plus précisément le premier module de générateur de tension 100 comprend un premier transformateur 11 associé au premier générateur de courant 10.

Avantageusement, le premier module de générateur de tension 100 comprend un premier modulateur configuré pour générer une troisième tension modulant la première tension. La troisième tension est préférentiellement une tension sinusoïdale. Le premier modulateur peut réaliser par exemple une modulation par modulation de largeur d'impulsion (MLI).

Le premier modulateur permet de moduler la première fréquence par une troisième fréquence. La troisième fréquence est strictement inférieure à la première fréquence. La troisième fréquence est comprise dans un troisième intervalle de fréquence étant préférentiellement inférieur au premier intervalle de fréquence. Le troisième intervalle de fréquence est préférentiellement compris entre 1 Hz et 150 Hz. Cet intervalle de fréquence s'entend comme des fréquences de type basses fréquences.

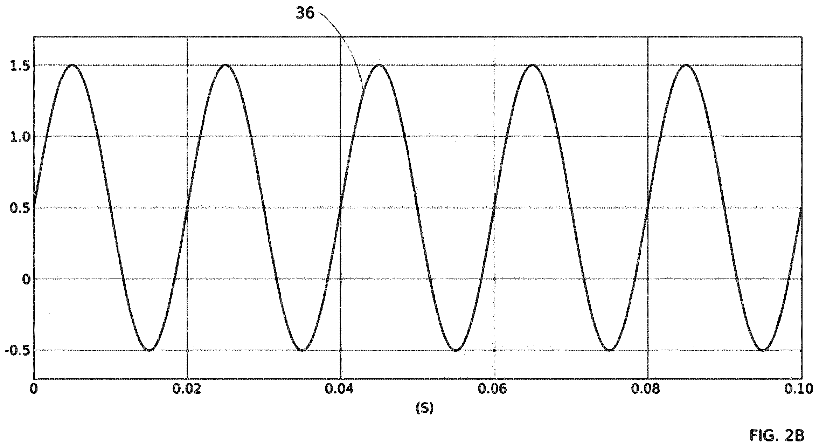

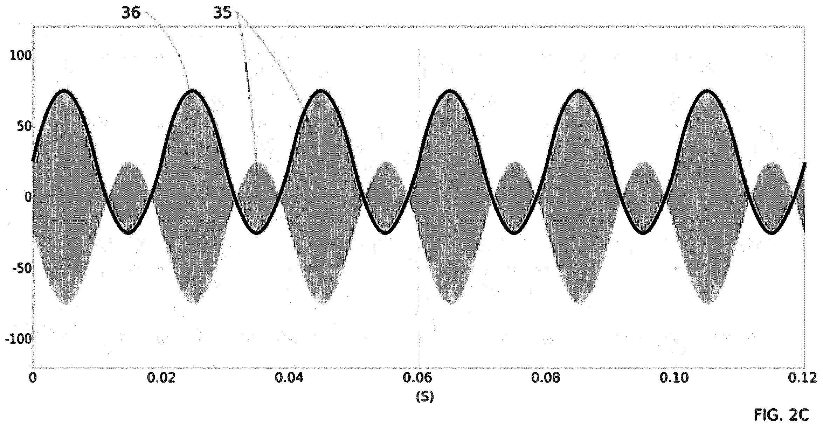

Selon une possibilité, la troisième fréquence est variable au cours du fonctionnement. Au cours du fonctionnement du premier module de générateur 100, la troisième fréquence peut varier pour faire varier la modulation de la première fréquence en fonction notamment du besoin de l'utilisateur. Un exemple de signal unitaire de basses fréquences est illustré à la figure 2B. Le signal est représenté avec un facteur de multiplication, en ordonnée, compris entre 0 et 1,5. Avantageusement, la sinusoïde est directement créée à partir du découpage primaire. La modulation de largeur d'impulsion permet d'avoir une sinusoïde modulable en amplitude à une fréquence basse par exemple de l'ordre de 1 Hz à 150 Hz. Le résultat est une tension sinusoïdale de 1 kHz à 10 kHz modulé en amplitude à une fréquence de 1 Hz à 150 Hz. Un exemple d'une telle modulation est illustré à la figure 2C. En électrostimulation, la modulation du courant stimulateur permet d'éviter la tétanie des muscles excités.

Le premier module de générateur de tension 100 permet de stimuler des contractions musculaires en profondeur et confortables.

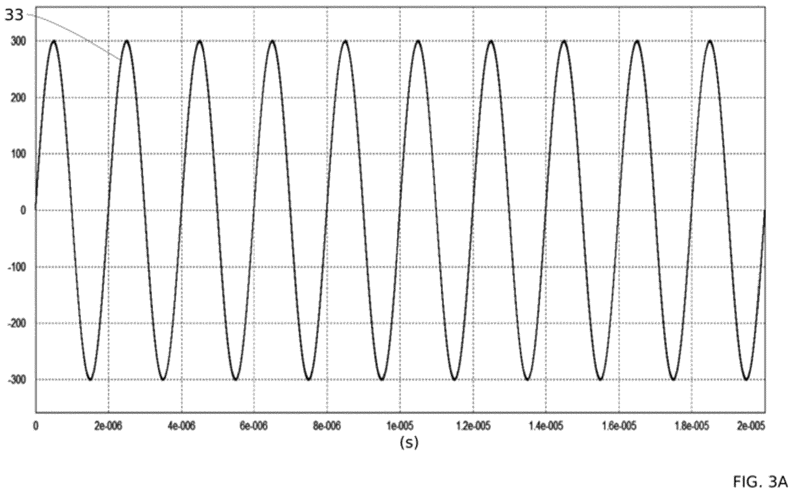

Le dispositif 1 comprend un deuxième module de générateur de tension 200 comprenant un deuxième générateur de tension 20. Le deuxième générateur de tension 20 est configuré pour générer une deuxième tension ayant une deuxième fréquence. Avantageusement, la deuxième tension est une tension sinusoïdale. La deuxième fréquence est strictement supérieure à la première fréquence. Préférentiellement, la deuxième fréquence est comprise dans un deuxième intervalle de fréquence strictement supérieur au premier intervalle. Préférentiellement, le deuxième intervalle est compris entre 100 kHz et 10 MHz, plus précisément de 100KHz à1,2 MHz. Cet intervalle de fréquence s'entend comme des fréquences de type hautes fréquences. Un exemple de tension de hautes fréquences est illustré à la figure 3A. Le deuxième module de générateur de tension 200 comprend une troisième borne de raccordement 22 et une quatrième borne de raccordement 23. Ce deuxième module générateur 200 est utilisé pour avoir des effets diathermiques. Ce type de courant résultant permet avantageusement de créer une accélération du métabolisme des tissus biologiques traversés.

Le générateur 200 fournit un courant avantageusement sinusoïdal de haute fréquence entre 10 kHz et 10MHz, plus précisément entre 100kHZ et 10 MHz, plus spécifiquement de 100 kHz à 1,2 MHz préférentiellement avec une impulsion de basse fréquence. La haute fréquence génère un effet diathermique sélectif et enfin la basse fréquence crée un effet anti-inflammatoire et anti-douleur.

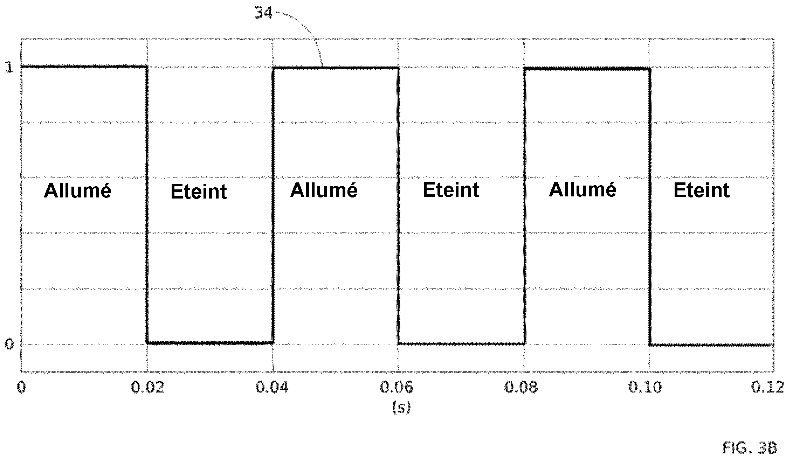

Avantageusement, le deuxième module de générateur de tension 200 comprend une commande d'activation 38 du générateur à la deuxième fréquence. La commande d'activation 38 est configurée pour commander la sortie du générateur à la deuxième fréquence par une quatrième fréquence à impulsion. La quatrième fréquence est strictement inférieure à la deuxième fréquence. La quatrième fréquence est comprise dans un quatrième intervalle de fréquence étant préférentiellement inférieur au deuxième intervalle de fréquence. Le quatrième intervalle de fréquence est préférentiellement compris entre 1 Hz et 150 Hz. Cet intervalle de fréquence s'entend comme des fréquences de type basses fréquences. Un exemple de basse fréquence à impulsion est illustré à la figure 3B. La quatrième fréquence est également dénommée fréquence carrée.

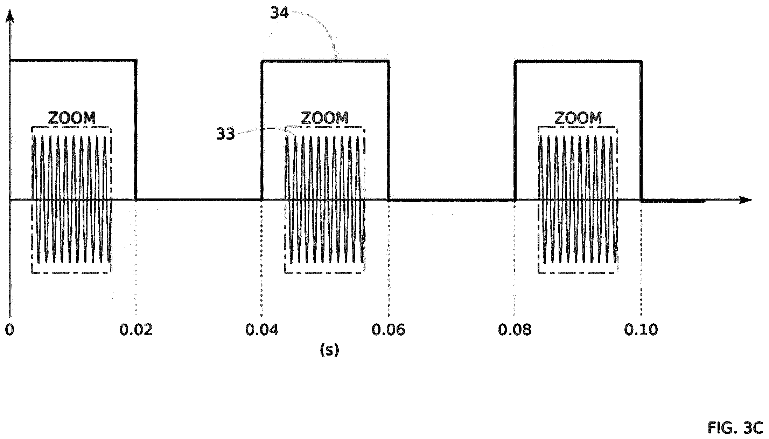

La tension de sortie du deuxième module de générateur de tension 200 est une tension sinusoïdale haute fréquence émise par impulsion à basse fréquence. Un exemple d'une modulation de ce type est illustré à la figure 3C.

Selon un mode de réalisation préféré, le dispositif, plus précisément le deuxième module de générateur de tension 200 comprend un deuxième transformateur 21 associé au deuxième générateur de tension 20.

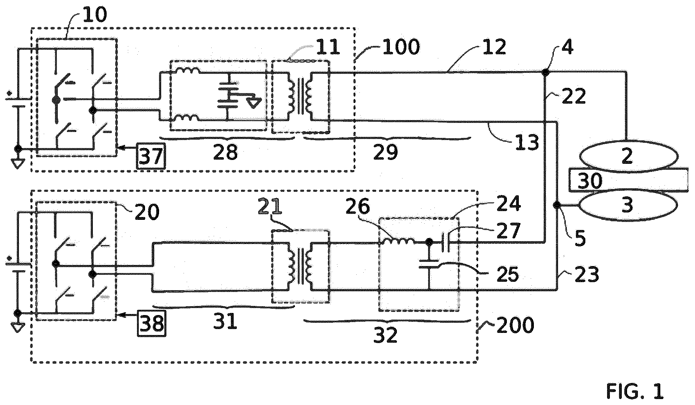

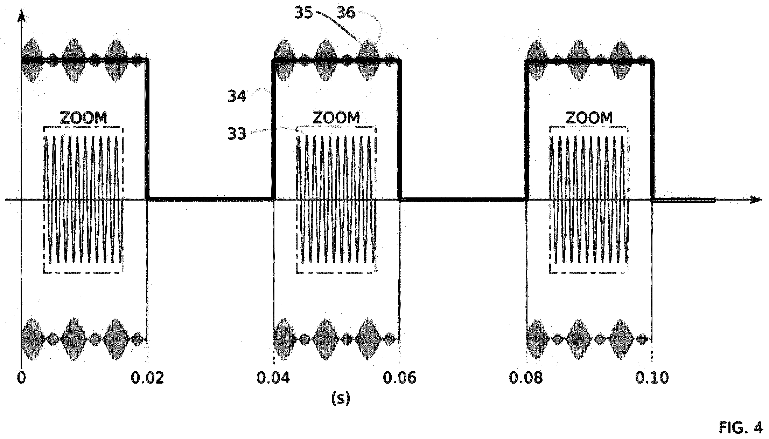

Avantageusement, le dispositif comprend un filtre de sortie 24 préférentiellement associé au deuxième transformateur 21. Le filtre de sortie 24 permet de filtrer au secondaire du deuxième transformateur 21. Le filtre de sortie 24 permet de créer une sinusoïde. Avantageusement, ce filtre comprend un circuit LC comprenant une bobine (L) 26 et un condensateur (C) 25. Préférentiellement, le filtre de sortie 24 comprend un condensateur résonant 27 permettant de résonner aux fréquences de fonctionnement. Le condensateur résonnant 27 est agencé en sortie de la bobine 26 et du condensateur 25. Un exemple de modulation obtenue en sortie du filtre de sortie 24 selon le circuit électrique illustré à la figure 1 est illustré à la figure 4. Selon ce mode de réalisation, la troisième borne de raccordement 22 et la quatrième borne de raccordement 23 sont agencées aux bornes du filtre de sortie 24.

Selon le présent enseignement, le dispositif 1 comprend un canal d'émission 4 et un canal de réception 5. Avantageusement, la première borne 12 et la troisième borne 22 sont reliées au même canal d'émission 4 tandis que la deuxième borne 13 et la quatrième borne 23 sont reliées au même canal de réception 5.

Préférentiellement, le canal d'émission 4 est destiné à être connecté à une électrode, préférentiellement une électrode active 2. L'électrode active 2 peut être capacitive et/ou résistive. Préférentiellement, le canal de réception 5 est destiné à être connecté à une électrode, préférentiellement une électrode neutre 3. L'électrode neutre 3 peut être fixe ou mobile.

Le dispositif selon la présente description est destiné à l'application d'une tension électrique sur le corps d'un utilisateur 30. Avantageusement, l'électrode active 2 est appliquée sur le corps de l'utilisateur 30 pour y fournir un courant tandis que l'électrode neutre 3 est également appliquée sur le corps de l'utilisateur 30 pour recevoir le courant fourni par l'électrode active 2 et ayant traversé une portion du corps de l'utilisateur 30.

Selon un mode de réalisation, la tension générée par le premier module de générateur 100 est également émise par impulsion à une fréquence égale à la quatrième fréquence générée par le deuxième module de générateur 200. A cet effet, le premier module de générateur 100 comprend avantageusement une commande d'émission 37 du signal de sortie du premier module de générateur 100 qui fonctionne par impulsion à la même fréquence que la quatrième fréquence de la commande d'activation 38 du deuxième module de générateur 200.

Le présent enseignement permet la combinaison de ces deux générateurs 10, 20 pour fournir au canal d'émission 4 un courant sinusoïdal moyenne fréquence modulé basse fréquence avec un courant sinusoïdal haute fréquence comme illustré en figure 4.

Le présente description propose ainsi de combiner au sein d'un unique et même canal d'émission 4, les vertus de l'électrostimulation sur la contraction musculaire grâce à des courants de basses fréquences et les vertus de la radiofréquence générant de la diathermie grâce à des courants de hautes fréquences.

Pour réussir à mixer les deux courants dans le même canal d'émission 4 sans perturber leur qualité, le présent enseignement propose de créer un courant, avantageusement sinusoïdal, de moyenne fréquence afin de porter, le signal de basse fréquence destiné à l'électrostimulation et de le combiner avec un courant de haute fréquence, avantageusement sinusoïdal, préférentiellement à impulsion basse fréquence, destiné à la diathermie. L'invention produit avantageusement un courant non invasif stimulant les mécanismes de cicatrisation naturels du corps et favorisant l'échange cellulaire. Ceci offre d'excellents résultats de réadaptation grâce à une récupération rapide des fonctions musculaires et articulaires.

Les applications possibles sont une diminution de la douleur, une élimination des tensions mécaniques, une stimulation de la circulation sanguine et lymphatique et un renforcement musculaire en profondeur et/ou une accélération de la régénération naturelle des tissus lésés ou abîmés.

Selon le présent enseignement, le premier module générateur 100 et le deuxième module générateur 200 génèrent respectivement une tension de manière simultanée.

On entend par simultané que le premier module de générateur de tension 100 génère une première tension émise par le canal d'émission 4 et pendant au moins une période de temps donnée non nulle, le deuxième module générateur de tension 200 génère une deuxième tension émise par le même canal d'émission 4. Pendant au moins cette période de temps donnée non nulle, l'électrode active 2 fournit le premier courant et le deuxième courant simultanément, par exemple à l'utilisateur 30.

Le dispositif comprend une unité de commande configurée pour contrôler la génération de la tension par le premier module générateur 100 et la génération de la tension par le deuxième module générateur 200. Avantageusement, l'unité de commande est également configurée pour commander l'intensité, la tension, l'inductance des courants générés et/ou l'impédance du dispositif.

Selon un mode de réalisation préféré, le premier transformateur 11 présente une première inductance et le deuxième transformateur 21 présente une deuxième inductance, le rapport de la première inductance et de la deuxième inductance est avantageusement supérieur ou égal à 500, préférentiellement supérieur ou égal à 1000. À titre d'exemple, le premier transformateur 11 est à 590mH maximum et le deuxième transformateur 21 est à 385µH minimum.

L'impédance du deuxième module de générateur est négligeable devant celle de l'utilisateur 30. Le chemin principal du courant est ainsi au travers de l'utilisateur 30.

Lorsque le deuxième générateur 20 est actif, le premier transformateur 11 ne modifie pas la tension sinusoïdale du deuxième transformateur 21, du fait de l'inductance, il n'y a pas d'interférence des fréquences.

Le présent enseignement concerne également un procédé de fonctionnement d'un dispositif tel que décrit ci-dessus, comprenant les étapes de fonctionnement suivantes :
- Une étape a. de génération d'une première tension ayant une première fréquence préférentiellement comprise dans un premier intervalle de fréquence par le premier module générateur de tension 100.
- Une étape b. de génération d'une deuxième tension ayant une deuxième fréquence strictement supérieure à la deuxième fréquence et préférentiellement comprise dans un deuxième intervalle de fréquence strictement supérieur au premier intervalle de fréquence par le deuxième module de générateur de courant 200.

Le procédé est configuré pour que les étapes a et b soient simultanées.

En figure 1, le circuit électrique du dispositif 1 selon le présent enseignement comprend un premier module de générateur 100 et un deuxième module de générateur 200. Le premier module de générateur 100 comprend un générateur de tension 10 et un premier transformateur 11. Le générateur de tension 10 comprend une topologie demi-pont ou pont complet permettant de générer un signal. Le générateur de tension 10 est avantageusement un générateur de tension moyenne fréquence piloté en MLI (modulation à largeur d'impulsion). Le premier module de générateur 100 comprend une première borne de raccordement 12 et une deuxième borne de raccordement 13.

Le premier transformateur 11 est connecté aux bornes du premier générateur 10. Le premier transformateur 11 comprend selon un mode de réalisation non limitatif un premier circuit primaire 28 comprenant avantageusement un filtre moyenne fréquence avantageusement par MLI et un premier circuit secondaire 29. Le filtre moyenne fréquence inférieur à 10 kHz permet de générer une sinusoïde à partir d'un signal carré. L'utilisation de la MLI permet d'avoir un filtrage optimisé. La première borne de raccordement 12 et la deuxième borne de raccordement 13 sont agencées au circuit secondaire 29 du premier transformateur 11.

Le deuxième module de générateur 200 comprend un deuxième générateur de tension 20 et un deuxième transformateur 21. Le générateur de tension 20 comprend une topologie demi-pont ou pont complet permettant de générer un signal créneau. Le deuxième module de générateur 200 comprend une troisième borne de raccordement 22 et une quatrième borne de raccordement 23. Le deuxième transformateur 21 est connecté aux bornes du deuxième générateur 20. Le deuxième transformateur 21 comprend selon un mode de réalisation non limitatif un deuxième circuit primaire 31 et un deuxième circuit secondaire 32. Préférentiellement, comme illustré ici, le dispositif comprend un filtre de sortie 24 connecté aux bornes du secondaire du deuxième transformateur 21. Le filtre de sortie 24 comprend une bobine, 26, une résistance 25 et condensateur résonnant 27. La troisième borne de raccordement 22 et la quatrième borne de raccordement 23 sont agencées sur le filtre de sotie 24. Le filtre de sortie est un filtre haute fréquence. Le filtre haute fréquence supérieur à 100 kHz permettant de générer une sinusoïde à partir d'un signal carré. Avantageusement, la structure bobine 26 - résistance 25 et un condensateur de résonnance 27 permet d'améliorer la forme de la sinusoïde par résonance préférentiellement à une fréquence f0. À titre d'exemple préféré, f0 est réglé à 400 kHz.

La première borne 11 et la troisième borne 21 sont reliées au canal d'émission 4 et la deuxième borne 13 et la quatrième borne 23 sont reliées au canal de réception 5.

Préférentiellement, tel qu'illustré, le canal d'émission 4 est connecté à au moins une électrode active 2 et le canal de réception 5 est connecté à au moins une électrode neutre 3. Les électrodes 2, 3 sont appliquées sur l'utilisateur 30.

Le générateur de tension 10 et le générateur de tension 20 sont isolés.

Les figures 2A à 2C illustrent la tension générée par le premier module de générateur 100.

La figure 2A illustre la tension à la première fréquence, ici 4 kHz, comprise dans le premier intervalle de fréquence correspondant aux moyennes fréquences. La tension est une tension sinusoïdale, courbe 35.

La figure 2B illustre le signal modulant à la deuxième fréquence, ici 50 Hz, comprise dans le troisième intervalle de fréquence correspondant aux basses fréquences. Le signal est un signal sinusoïdal, courbe 36.

Le premier module de générateur de tension 100 génère ainsi la tension sinusoïdale illustrée à la figure 2C, présentant une moyenne fréquence à 4 kHz, courbe 35, modulée en amplitude à 150% par des basses fréquences, courbe 36. La modulation se fait à 100% puis à 50% par la fréquence de modulation basses fréquences.

Les figures 3A à 3C illustrent la tension générée par le deuxième module de générateur 200.

La figure 3A illustre la tension à la deuxième fréquence, ici 500 kHz, comprise dans le deuxième intervalle de fréquence correspondant aux hautes fréquences. La tension est une tension sinusoïdale, courbe 33.

La figure 3B illustre le signal modulant à la quatrième fréquence, ici 25 Hz, comprise dans le quatrième intervalle de fréquence correspondant aux basses fréquences. Le signal est un signal carré. Le signal modulant est ainsi un pulse, courbe 34.

Le deuxième module de générateur de tension 200 génère ainsi la tension sinusoïdale illustrée à la figure 3C présentant une haute fréquence à 500 kHz modulée par des basses fréquences de type carré. Un détail de tension sinusoïdale illustrée à la figure 3A est représenté dans les carrés de la figure 3C. On retrouve ainsi la tension haute fréquence qui est modulée par les pulses basses fréquences.

En figure 4, il est représenté la tension sinusoïdale en sortie du canal d'émission 4 lors d'une application simultanée de deux tensions. La tension sinusoïdale obtenue est la somme de la première tension générée par le premier module de générateur 100 et de la deuxième tension générée par le deuxième module de générateur 200. Le signal de sortie dans le canal d'émission 4 est une tension sinusoïdale présentant des hautes fréquences et des moyennes fréquences modulées par des basses fréquences. Ici, la première tension générée par le premier module de générateur 100 est également modulée par la basse fréquence à impulsion du deuxième module de générateur 200. Pour cela comme décrit ci-dessus, le dispositif comprend une commande d'émission 37 de la tension émise configurée pour émettre la tension du premier module de générateur à la quatrième fréquence. On retrouve donc ici sur la fréquence basse des pulses (courbe 34), la haute fréquence (courbe 33) et la moyenne fréquence (courbe 35) modulée basse fréquence (courbe 36) en amplitude supérieure à 100%.

Le présent enseignement n'est pas limité aux modes de réalisations précédemment décrits et s'étend à tous les modes de réalisation couverts par la demande.

### Liste des références

- 1.: Dispositif
- 2.: Électrode active
- 3.: Électrode neutre
- 4.: Canal d'émission
- 5.: Canal de réception
- 10.: Premier générateur
- 11.: Premier transformateur
- 12.: première borne de raccordement
- 13.: deuxième borne de raccordement
- 20.: Deuxième générateur
- 21.: Deuxième transformateur
- 22.: Troisième borne de raccordement
- 23.: Quatrième borne de raccordement
- 24.: Filtre de sortie
- 25.: Condensateur
- 26.: Bobine
- 27.: Condensateur résonnant
- 28.: Premier circuit primaire
- 29.: Premier circuit secondaire
- 30.: Utilisateur
- 31.: Deuxième circuit primaire
- 32.: Deuxième circuit secondaire
- 33.: courbe haute fréquence
- 34.: courbe basse fréquence à impulsion
- 35.: courbe porteuse à moyenne fréquence
- 36.: courbe basse fréquence
- 37.: commande d'émission
- 38.: commande d'activation
- 100.: Premier module générateur
- 200.: Deuxième module générateur
- 300.: Porteuse
- 400.: Période d'application
- 500.: Première période
- 600.: Deuxième période

La présente invention est définie par les revendications suivantes.

## Revendications

1. Dispositif d'électrothérapie (1) comprenant :
• un premier module générateur de tension (100) configuré pour générer une première tension ayant une première fréquence et comprenant une première borne de raccordement (12) et une deuxième borne de raccordement (13),
• un deuxième module générateur de tension (200) configuré pour générer une deuxième tension ayant une deuxième fréquence strictement supérieure à la première fréquence et comprenant une troisième borne de raccordement (22) et une quatrième borne de raccordement (23),
• un canal d'émission (4),
• un canal de réception (5),
dispositif dans lequel la première borne de raccordement (12) et la troisième borne de raccordement (22) sont reliées audit canal d'émission (4) et la deuxième borne de raccordement (13) et la quatrième borne de raccordement (23) sont reliées audit canal de réception (5), ledit dispositif comprenant en outre
• une unité de commande configurée pour simultanément :
a. commander le premier module générateur de tension (100) pour générer la première tension à une première fréquence,
b. commander le deuxième module générateur de tension (200) pour générer la deuxième tension à une deuxième fréquence,
l'unité de commande étant configurée pour générer dans le canal d'émission (4), un signal associant une tension sinusoïdale à la première fréquence modulée par une troisième fréquence et une tension sinusoïdale à la deuxième fréquence modulée par une quatrième fréquence, le signal étant la somme de la tension du premier module de générateur et de la tension du deuxième module de générateur,
la première fréquence étant comprise dans un premier intervalle de fréquence entre 1 kHz à 10 kHz,
la deuxième fréquence étant comprise dans un deuxième intervalle de fréquence compris entre 100 kHz à 10 MHz,
la troisième fréquence est comprise dans un troisième intervalle de fréquence compris entre 1 Hz et 150 Hz,
la quatrième fréquence est comprise dans un quatrième intervalle de fréquence compris entre 1 Hz et 150 Hz.

2. Dispositif selon la revendication précédente dans lequel le premier module générateur de tension (100) est configuré pour générer une tension sinusoïdale.

3. Dispositif selon l'une quelconque des revendications précédentes dans lequel le premier module générateur de tension (100) est configuré pour générer une tension sinusoïdale de première fréquence modulée par la troisième fréquence.

4. Dispositif selon l'une quelconque des revendications précédentes dans lequel le deuxième module générateur de tension (200) est configuré pour générer une tension sinusoïdale.

5. Dispositif selon l'une quelconque des revendications précédentes dans lequel le deuxième module générateur de tension (200) est configuré pour générer une tension sinusoïdale de deuxième fréquence modulé par la quatrième fréquence.

6. Dispositif selon l'une quelconque des revendications précédentes dans lequel la quatrième fréquence est à impulsion.

7. Dispositif selon l'une quelconque des revendications précédentes dans lequel le deuxième module de générateur de tension (200) comprend une commande d'activation (38) configurée pour générer une tension sinusoïdale à impulsion à la quatrième fréquence.

8. Dispositif selon l'une quelconque des revendications précédentes dans lequel le premier module de générateur de tension (100) comprend une commande d'émission (37) configurée pour émettre la tension du premier module de générateur (100) à la quatrième fréquence.

9. Dispositif d'électrothérapie selon l'une quelconque des revendications précédentes dans lequel le premier module de générateur (100) comprend un premier transformateur (11) présentant une première inductance et le deuxième module de générateur (200) comprend un deuxième transformateur (12) présentant une deuxième inductance, de préférence le rapport de la première inductance et de la deuxième inductance étant supérieur à 500, préférentiellement supérieure à 1000.

10. Dispositif d'électrothérapie selon l'une quelconque des revendications précédentes dans lequel le deuxième module de générateur (200) comprend un filtre de sortie (24) comprenant un circuit LC comprenant une bobine (26) et un condensateur (25).

11. Dispositif d'électrothérapie selon la revendication précédente dans lequel le filtre de sortie (24) comprend un condensateur résonant (27) permettant de résonner à la deuxième fréquence du deuxième module de générateur (200).

12. Dispositif d'électrothérapie selon l'une quelconque des revendications précédentes dans lequel l'unité de commande est configurée pour faire varier l'impédance dans le canal d'émission (4) lorsqu'elle commande le premier module générateur de tension (100) pour générer la première tension à une première fréquence.

## Patentansprüche

1. Elektrotherapievorrichtung (1), umfassend:
• ein erstes Spannungsgeneratormodul (100), das so konfiguriert ist, dass es eine erste Spannung erzeugt, die eine erste Frequenz aufweist, und eine erste Anschlussklemme (12) und eine zweite Anschlussklemme (13) umfasst,
• ein zweites Spannungsgeneratormodul (200), das so konfiguriert ist, dass es eine zweite Spannung erzeugt, die eine zweite Frequenz aufweist, die strikt über der ersten Frequenz liegt, und eine dritte Anschlussklemme (22) und eine vierte Anschlussklemme (23) umfasst,
• einen Sendekanal (4),
• einen Empfangskanal (5),
wobei bei der Vorrichtung die erste Anschlussklemme (12) und die dritte Anschlussklemme (22) mit dem Sendekanal (4) verbunden sind und die zweite Anschlussklemme (13) und die vierte Anschlussklemme (23) mit dem Empfangskanal (5) verbunden sind, wobei die Vorrichtung ferner umfasst
• eine Steuereinheit, die so konfiguriert ist, dass sie gleichzeitig:
a. das erste Spannungsgeneratormodul (100) so steuert, dass es die erste Spannung bei einer ersten Frequenz erzeugt, b. das zweite Spannungsgeneratormodul (200) so steuert, dass es die zweite Spannung bei einer zweiten Frequenz erzeugt,
wobei die Steuereinheit so konfiguriert ist, dass sie in dem Sendekanal (4) ein Signal erzeugt, das einer Sinusspannung mit der ersten Frequenz, moduliert durch eine dritte Frequenz, und einer Sinusspannung mit der zweiten Frequenz, moduliert durch eine vierte Frequenz, zuordnet, wobei das Signal die Summe der Spannung des ersten Generatormoduls und der Spannung des zweiten Generatormoduls ist,
wobei die erste Frequenz in einem ersten Frequenzbereich zwischen 1 kHz und 10 kHz liegt,
wobei die zweite Frequenz in einem zweiten Frequenzbereich zwischen 100 kHz und 10 MHz liegt,
die dritte Frequenz in einem dritten Frequenzbereich zwischen 1 Hz und 150 Hz liegt,
die vierte Frequenz in einem vierten Frequenzbereich zwischen 1 Hz und 150 Hz liegt.

2. Vorrichtung nach vorhergehendem Anspruch, wobei das erste Spannungsgeneratormodul (100) so konfiguriert ist, dass es eine sinusförmige Spannung erzeugt.

3. Vorrichtung nach einem der vorhergehenden Ansprüche, wobei das erste Spannungsgeneratormodul (100) so konfiguriert ist, dass es eine sinusförmige Spannung erster Frequenz, moduliert durch die dritte Frequenz, erzeugt.

4. Vorrichtung nach einem der vorhergehenden Ansprüche, wobei das zweite Spannungsgeneratormodul (200) so konfiguriert ist, dass es eine sinusförmige Spannung erzeugt.

5. Vorrichtung nach einem der vorhergehenden Ansprüche, wobei das zweite Spannungsgeneratormodul (200) so konfiguriert ist, dass es eine sinusförmige Spannung zweiter Frequenz, moduliert durch die vierte Frequenz, erzeugt.

6. Vorrichtung nach einem der vorhergehenden Ansprüche, wobei die vierte Frequenz impulsartig ist.

7. Vorrichtung nach einem der vorhergehenden Ansprüche, wobei das zweite Spannungsgeneratormodul (200) einen Aktivierungsbefehl (38) umfasst, der so konfiguriert ist, dass er eine sinusförmige Impulsspannung bei der vierten Frequenz erzeugt.

8. Vorrichtung nach einem der vorhergehenden Ansprüche, wobei das erste Spannungsgeneratormodul (100) eine Sendesteuerung (37) umfasst, die so konfiguriert ist, dass sie die Spannung des ersten Generatormoduls (100) bei der vierten Frequenz sendet.

9. Elektrotherapievorrichtung nach einem der vorhergehenden Ansprüche, wobei das erste Generatormodul (100) einen ersten Transformator (11) umfasst, der eine erste Induktivität aufweist, und das zweite Generatormodul (200) einen zweiten Transformator (12) umfasst, der eine zweite Induktivität aufweist, wobei das Verhältnis der ersten Induktivität zu der zweiten Induktivität vorzugsweise größer als 500, vorzugsweise größer als 1000, ist.

10. Elektrotherapievorrichtung nach einem der vorhergehenden Ansprüche, wobei das zweite Generatormodul (200) einen Ausgangsfilter (24) umfasst, der eine LC-Schaltung umfasst, die eine Spule (26) und einen Kondensator (25) umfasst.

11. Elektrotherapievorrichtung nach vorhergehendem Anspruch, wobei der Ausgangsfilter (24) einen Resonanzkondensator (27) umfasst, der es ermöglicht, mit der zweiten Frequenz des zweiten Generatormoduls (200) zu schwingen.

12. Elektrotherapievorrichtung nach einem der vorhergehenden Ansprüche, wobei die Steuereinheit so eingerichtet ist, dass sie die Impedanz in dem Sendekanal (4) variiert, wenn sie das erste Spannungsgeneratormodul (100) steuert, um die erste Spannung bei einer ersten Frequenz zu erzeugen.

## Claims

1. An electrotherapy device (1) comprising:
• a first voltage generator module (100) configured to generate a first voltage having a first frequency and comprising a first connection terminal (12) and a second connection terminal (13),
• a second voltage generator module (200) configured to generate a second voltage having a second frequency strictly higher than the first frequency and comprising a third connection terminal (22) and a fourth connection terminal (23),
• a transmission channel (4),
• a reception channel (5),
in which device the first connection terminal (12) and the third connection terminal (22) are connected to said transmission channel (4), and the second connection terminal (13) and the fourth connection terminal (23) are connected to said reception channel (5), said device further comprising
• a control unit configured to simultaneously:
a. control the first voltage generator module (100) to generate the first voltage at a first frequency,
b. control the second voltage generator module (200) to generate the second voltage at a second frequency,
the control unit being configured to generate in the transmission channel (4) a signal associating a sinusoidal voltage with the first frequency modulated by a third frequency and a sinusoidal voltage with the second frequency modulated by a fourth frequency, the signal being the sum of the voltage of the first generator module and the voltage of the second generator module,
the first frequency being comprised in a first frequency interval between 1 kHz and 10 kHz,
the second frequency being comprised in a second frequency interval between 100 kHz and 10 MHz,
the third frequency is comprised in a third frequency interval between 1 Hz and 150 Hz,
the fourth frequency is comprised in a fourth frequency interval between 1 Hz and 150 Hz.

2. The device according to the preceding claim, wherein the first voltage generator module (100) is configured to generate a sinusoidal voltage.

3. The device according to any one of the preceding claims, wherein the first voltage generator module (100) is configured to generate a sinusoidal voltage with the first frequency modulated by the third frequency.

4. The device according to any one of the preceding claims, wherein the second voltage generator module (200) is configured to generate a sinusoidal voltage.

5. The device according to any one of the preceding claims, wherein the second voltage generator module (200) is configured to generate a sinusoidal voltage with the second frequency modulated by the fourth frequency.

6. The device according to any one of the preceding claims, wherein the fourth frequency is pulsed.

7. The device according to any one of the preceding claims, wherein the second voltage generator module (200) comprises an activation control (38) configured to generate a pulse sinusoidal voltage at the fourth frequency.

8. The device according to any one of the preceding claims, wherein the first voltage generator module (100) comprises a transmission control (37) configured to transmit the voltage of the first generator module (100) at the fourth frequency.

9. The electrotherapy device according to any one of the preceding claims, wherein the first generator module (100) comprises a first transformer (11) having a first inductance and the second generator module (200) comprises a second transformer (12) having a second inductance, preferably the ratio of the first inductance to the second inductance being greater than 500, preferably greater than 1000.

10. The electrotherapy device according to any one of the preceding claims, wherein the second generator module (200) comprises an output filter (24) comprising an LC circuit comprising a coil (26) and a capacitor (25).

11. The electrotherapy device according to the preceding claim, wherein the output filter (24) comprises a resonating capacitor (27) for resonating at the second frequency of the second generator module (200).

12. The electrotherapy device according to any one of the preceding claims, wherein the control unit is configured to vary the impedance in the transmission channel (4) when it controls the first voltage generator module (100) to generate the first voltage at a first frequency.
